**Europäisches Patentamt**

**European Patent Office**

**Office européen des brevets**

(11) Veröffentlichungsnummer : **0 341 488 B1**

(12) **EUROPÄISCHE PATENTSCHRIFT**

(45) Veröffentlichungstag der Patentschrift :
**24.06.92 Patentblatt 92/26**

(51) Int. Cl.⁵ : **A61M 5/14**

(21) Anmeldenummer : **89107524.4**

(22) Anmeldetag : **26.04.89**

(54) **Druckmembrananordnung für Infusionen.**

(30) Priorität : **11.05.88 DE 3816128**

(43) Veröffentlichungstag der Anmeldung :
**15.11.89 Patentblatt 89/46**

(45) Bekanntmachung des Hinweises auf die
Patenterteilung :
**24.06.92 Patentblatt 92/26**

(84) Benannte Vertragsstaaten :
**AT BE CH DE FR GB LI LU NL SE**

(56) Entgegenhaltungen :
**EP-A- 0 248 632**
**WO-A-86/07266**
**DE-A- 3 209 721**

(73) Patentinhaber : **Mokros, Klaus, Dipl.-Ing.**
**Brentanostrasse 51**
**W-8755 Alzenau (DE)**

(72) Erfinder : **Mokros, Klaus, Dipl.-Ing.**
**Brentanostrasse 51**
**W-8755 Alzenau (DE)**

(74) Vertreter : **Quermann, Helmut, Dipl.-Ing.**
**Postfach 6145 Gustav-Freytag-Strasse 25**
**W-6200 Wiesbaden (DE)**

EP 0 341 488 B1

**Beschreibung**

Die Erfindung betrifft eine Druckmembrananordnung für Infusionen, gemäß dem ersten Teil von Anspruch 1.

Druckwarnsysteme für Infusionen dienen dem Zweck, unzulässige Drucke im Infusionssystem erkennen zu können, so daß gesundheitsschädigende Folgen für die Patienten ausgeschlossen sind. So kann beispielsweise während des Infusionsvorganges der Katheter verstopfen oder es ist der Katheter parageschoben, so daß sich bei der fortwährenden Förderung der Infusionsflüssigkeit mittels der Druckpumpe erhöhte Drücke in der zum Katheter führenden Infusionsleitung einstellen, ohne daß Infusionsflüssigkeit dem Patienten zugeführt wird. Handelt es sich beispielsweise um blutdruckregelnde Mittel, die zuzuführen sind, ist leicht einsehbar, daß eine Unterbrechung der Zufuhr unmittelbar den Regelungsmechanismus des Körpers negativ beeinflußt. Es muß daher von einem Druckwarnsystem für Infusionen gefordert werden, daß es innerhalb kürzester Zeit nach Auftreten des erhöhten Druckes ein auf dieses Ereignis hinweisendes Signal abgibt.

Eine Druckmembrananordnung ist aus der DE-A1-3 209 721 bekannt. Bei dieser ist der Druckgeber als Manometer ausgebildet, das mit einer Membran versehen ist, die sich in Abhängigkeit vom Druck der Infusionsflüssigkeit durchbiegt. Bei Erreichen eines bestimmten, vorgebbaren Grenzwertes betätigt die Membran einen Druckaufnehmer in Art eines Schalters, der einerseits eine optische oder akustische Alarmvorrichtung betätigt, andererseits die Infusionspumpe ausschaltet. Der Aufbau des Manometers sowie das Zusammenwirken des Manometers mit der Schaltvorrichtung ist nicht näher beschrieben.

Aus der WO-A1-86/07266 ist eine Druckmembrananordnung gemäß dem ersten Teil von Anspruch 1 bekannt, bei der die Membran sackförmig und elastisch ausgebildet ist. Bei Unterschreiten eines definierten Druckes in der Druckleitung ist die Membran in das zugeordnete Leitungssegment der Druckleitung eingestülpt und schnappt bei einer definierten Druckerhöhung aus dem Leitungssegment heraus in den Wirkweg eines optischen Sensors, der das hierdurch erzeugte Signal an den Signalgeber weitergibt. Zur Verstellung der Empfindlichkeit des Druckaufnehmers ist es dort erforderlich, jeweils entsprechend der Schnappcharakteristik der Membran eine bestimmte Membran definierter Elastizität bzw. Stärke auszuwählen. Für eine praktikable Anwendung der Druckmembrananordnung wäre es allerdings Voraussetzung, den Fließdruck in der Infusionsleitung zu kennen, um so die Druckmembrananordnung an den Patienten anpassen zu können.

Es ist Aufgabe der vorliegenden Erfindung, eine Druckmembrananordnung für Infusionen der genannten Art zu schaffen, mit der systemunabhäng eine Druckerhöhung in der Infusionsleitung unmittelbar festgestellt werden kann, wobei die Sensibilität des Druckaufnehmers variierbar sein soll.

Gelöst wird die Aufgabe durch die in Anspruch 1 angegebene Druckmembrananordnung.

Die dehnbare Membran steht über den ihr zugeordneten Arbeitsraum in unmittelbarer Verbindung mit der Druckleitung. Hierdurch bewirkt eine Druckerhöhung in der Druckleitung ein Aufblasen, das heißt Ausdehnen der Membran, wobei diese Dehnbewegung dazu benutzt wird, den Druckaufnehmer zu betätigen. Baulich besonders einfach gestaltet sich der Druckaufnehmer, wenn er einen Anlegeteller für die Membran des Druckgebers aufweist, wobei der Anlegeteller entgegen der Ausdehnrichtung der Membranfeder beaufschlagt sein sollte. Ist im Grundkörper des Druckaufnehmers im Wirkweg des Anlegetellers der Druckschalter oder Sensor angeordnet, kann die Ausdehnbewegung der Membran bei Verwendung nur weniger Bauteile unmittelbar über den Anlegeteller auf den Druckschalter oder Sensor übertragen werden. Die Aufnahme des Druckschalters bzw. Sensor in der relativ zum Grundkörper des Druckaufnehmers verstellbaren Halterung ermöglicht es, die Sensibilität des Druckschalters bzw. Sensors zu variieren.

Besonders einfach läßt sich der Druckgeber mit dem Druckaufnehmer dadurch verbinden, daß im Grundkörper des Druckaufnehmers eine Öffnung vorgesehen ist, in die der Druckgeber einsetzbar und in der er mittels der Haltefeder gehalten ist. Im einzelnen wird es als zweckmäßig angesehen, wenn der Grundkörper des Druckgebers eine Ringöffnung zur Aufnahme der dann kreisförmig ausgebildeten Membran aufweist.

Die erfindungsgemäße Druckmembrananordnung für Infusionen mißt somit über einen in einer separaten Druckleitung angeordneten Druckgeber den Druck im Infusionssystem. Dies bedeutet, daß die Druckleitung nicht von der Infusionsflüssigkeit durchflossen ist und bei ordnungsgemäß arbeitendem Infusionssystem der Druckgeber stets deaktiviert ist. Insbesondere bei den für Infusionszwecke Verwendung findenden Leitungen, die geringste Durchmesser aufweisen, wird in aller Regel die Infusionsflüssigkeit nur geringfügig in die Druckleitung eindringen, da deren der Infusionsleitung abgewandtes Ende mittels des Druckgebers verschlossen ist.

Vorteilhaft ist die Druckleitung im Bereich des Katheters mit der Infusionsleitung verbunden. Es ist hierdurch sichergestellt, daß beispielsweise ein Verschluß des Katheters in dessen unmittelbarer Nachbarschaft durch den Druckgeber festgestellt werden kann. Eine einfache Verbindungsmöglichkeit von Druckleitung und Infusionsleitung ergibt sich, wenn ein T-Kupplungsstück zu deren Verbindung vorgesehen ist. Es ist dabei nur nötig, die Druckleitung auf einen Anschluß des T-Kupplungsstückes aufzustecken, ohne Rücksicht darauf, ob

2

sich in der Druckleitung Flüssigkeit oder Luft befindet, da eine Druckerhöhung ohnehin nur dazu führen würde, daß Infusionsflüssigkeit von der Infusionsleitung in die Druckleitung gedrückt wird und bei einer eventuellen Druckreduzierung nur ein so geringer Eintritt von Luft in die Infusionsleitung zu verzeichnen wäre, der keine Gesundheitsgefährdung des Patienten zur Folge hätte.

In den Figuren ist die Erfindung an einer Ausführungsform beispielsweise dargestellt, ohne auf diese Ausführungsform beschränkt zu sein. Es zeigt:

Figur 1 eine schematische Darstellung der wesentlichen Teile der Druckmembrananordnung für Infusionen,

Figur 2 eine Ansicht der Breitseite des Druckaufnehmers und der Druckleitung mit der Verbindung zur Infusionsleitung,

Figur 3 eine der Figur 2 entsprechende Ansicht jedoch in Richtung der Schmalseite des Druckgebers gesehen,

Figur 4 einen Längsmittelschnitt durch den Druckaufnehmer mit in diesem eingesetzten Druckgeber,

Figur 5 eine Ansicht der Breitseite des Signalgebers und

Figur 6 eine Ansicht der Schmalseite des Signalgebers.

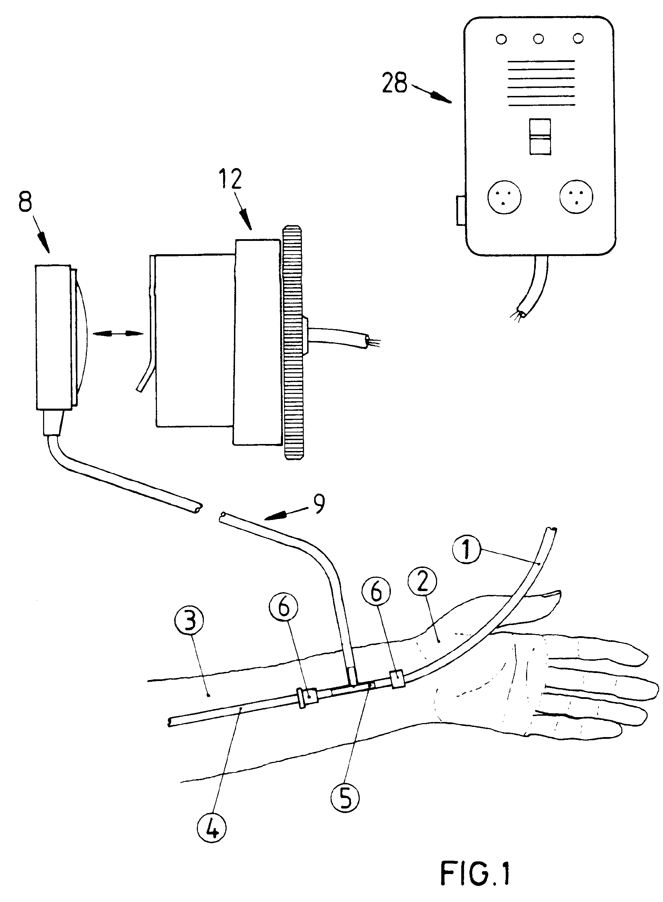

Die Figur 1 zeigt eine mit einem Infusionsflüssigkeit enthaltenden, nicht näher dargestellten Behältnis verbundene Infusionsleitung 1, die nur im Bereich einer Hand 2 dargestellt ist. In den Unterarm 3 ist in eine nicht näher gezeigte Vene ein Katheter 4 geschoben, dieser ist mit der Infusionsleitung 1 über ein T-Stück 5 verbunden, das Luer Lock-Konnektoren Male/Female aufweist. Die Infusionsflüssigkeit kann somit durch die Infusionsleitung 1 und den durchgehenden Ast des T-Stückes 5 in den Katheter 4 und damit in die Vene des Körpers gelangen, wobei es unter dem Aspekt der vorliegenden Erfindung unerheblich ist, ob die Infusionsflüssigkeit mittels einer Infusionspumpe oder nur von einer Infusionsflasche direkt der Infusionsleitung 1 zugeführt wird.

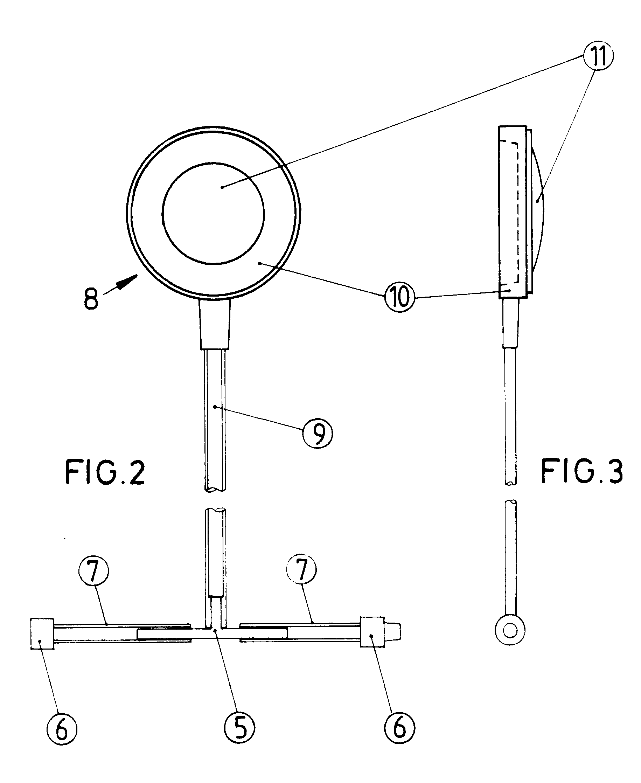

Figur 2 zeigt im Detail die Verbindung des T-Stückes 5 mit den jeweiligen Konnektoren 6 über Schlauchanschlüsse 7. Darüber hinaus verdeutlichen die Figuren 2 und 3 den Anschluß des Druckgebers 8, der über eine eine Einheit mit dem T-Stück bildende Druckleitung 9 in offener Verbindung mit der Infusionsleitung 1 und dem Katheter 4 steht. Der Druckgeber 8 weist einen Grundkörper 10 auf, mit einer Ringöffnung zur Aufnahme einer kreisförmigen Membran 11, die aufblas- und dehnbar ist. Vor dem Anlegen der Infusion wird die Einheit zwischen Katheter 4 und Ende der Infusionsleitung 1 mit den vorhandenen Luer-Lock-Konnektoren adaptiert. In der relativ kurzen Druckleitung 9 und in dem zwischen dem Grundkörper 10 des Druckgebers 8 und der Membran 11 verbleibenden Raum befindet sich Luft und zwar aufgrund der Abmessungen des Luftraumes mit einem Gesamtvolumen von weniger als 1 ml, die während der Infusion dort quasi stationär verbleibt. Sollte ein Verschluß des Katheters 4 erfolgen, führt dies zu einer Erhöhung des Druckes im Infusionssystem und damit der im T-Stück 5 und der Infusionsleitung 1 befindlichen Infusionsflüssigkeit mit der Folge, daß die in der Druckleitung 9 und dem Druckgeber 8 befindliche Luft komprimiert wird und sich hierdurch die Membran 11 infolge Ausdehnung stärker nach außen wölbt. Eine Wölbung wäre dabei selbstverständlich auch dann zu verzeichnen, wenn sich innerhalb der Druckleitung 9 und des Druckgebers 8 Flüssigkeit befinden würde, bei einer entsprechend empfindlich ausgebildeten Membran 11 würde die Druckhöhe einer oberhalb des Katheters aufgehängten Infusionsflasche vollständig ausreichen, daß die Membran 11 im erforderlichen Umfang gedehnt würde, was verdeutlicht, daß die erfindungsgemäße Ausbildung der Druckmembrananordnung für Infusionen nicht auf Druckpumpeninfusionen beschränkt ist.

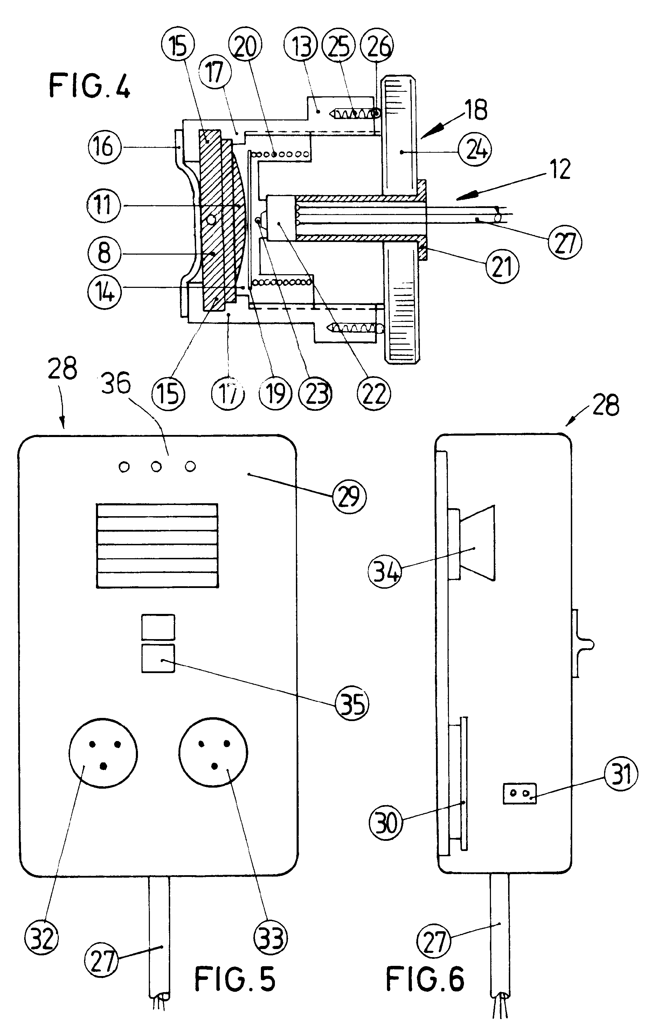

Die Figur 4 zeigt das Zusammenwirken des Druckgebers 8 mit einem Druckaufnehmer 12. Dieser weist gleichfalls einen Grundkörper 13 auf, mit einer Öffnung 14 mit zwei parallelen Nuten 15, in die der Grundkörper 10 des Druckgebers 8 einsteckbar ist, wobei eine mit dem Grundkörper 13 verbundene Haltefeder 16 den Druckgeber 8 in eine definierte Stellung gegen Anschläge 17 im Grundkörper 13 drückt. In den Grundkörper 13 ist eine Stellschraube 18 eingeschraubt, wobei sich ein Anlegeteller 19 für die Membrane 11 über eine Druckfeder 20 an der Stellschraube 18 abstützt, die in einer zentrischen Bohrung drehfest eine verstellbare Halterung 21 für einen Druckschalter 22 aufweist, dessen Druckstift 23 in geringfügigem Abstand von dem Anlegeteller 19 und der entspannten Membran 11 angeordnet ist. Schließlich ist drehfest mit der Stellschraube 18 ein Stellring 24 für die Einstellung der Empfindlichkeit des Druckaufnehmers verbunden, der auf seiner dem Grundkörper 13 zugewandten Fläche mit einer Vielzahl von nicht näher dargestellten Ausnehmungen versehen ist, in die mittels Druckfedern 25 vorgespannte Rastkugeln 26 einführbar sind. Eine Ausdehnung der Membrane 11 bei erhöhtem Druck in der Druckleitung 9 führt somit zum Anlegen der Membran an den Anlegeteller 19 und dessen Verschiebung entgegen der Kraft der Druckfeder 20, ist ein bestimmter Verschiebeweg des Anlegetellers 19 erreicht, wird der Druckstift 23 berührt und entsprechend der Druckschalter 22 betätigt.

Die Figur 4 zeigt ein mit dem Druckschalter 22 verbundendes, dreipoliges Anschlußkabel für den in den Figuren 5 und 6 gezeigten Signalgeber 28. Dieser besteht im wesentlichen aus einem Gehäuse 29, einer in diesem befindlichen Elektronik 30 zur Signalverarbeitung, einem Anschluß 31 für ein Netzgerät, einem Ausgang 32 für Schwesternruf, einem Ausgang 33 für andere Alarmgeber oder Computersignal, einem Lautsprecher bzw. einer Hupe 34, dem Anschlußkabel 27 zum Druckaufnehmer 12, einem Ein/Aus Schalter 35 sowie Leuchtdioden 37 zum Anzeigen des Betriebszustandes des Gerätes.

**Patentansprüche**

1. Druckmembrananordnung für Infusionen, insbesondere für mittels einer Infusionspumpe zugeführte Infusionen, mit einer von dem die Infusionsflüssigkeit enthaltenden Behältnis zum Katheter führenden Infusionsleitung (1), einer mit der Infusionsleitung (1) verbundenen, geschlossenen Druckleitung (9), mit deren Druckraum ein Druckgeber (8) verbunden ist, der eine dehnbare Membran (11) aufweist, wobei die Membran (11) mit einem Druckaufnehmer (12) zusammenwirkt, über den ein Signalgeber (28) ansteuerbar ist, und wobei der Druckgeber (8) einen Grundkörper (10) zur Halterung der Membran (11) aufweist, welcher in einen Grundkörper (13) des Druckaufnehmers (12) einsetzbar ist, **dadurch gekennzeichnet**, daß der Grundkörper (10) des Druckgebers (8) mittels einer Haltefeder (16) im Grundkörper (13) des Druckaufnehmen (12) gehalten ist, und daß ein Druckschalter (22) oder Sensor in einer verstellbaren Halterung (18, 24) gehalten ist, die vom grundkörper (13) des Druckaufnehmers (12) aufgenommen wird, wobei die Halterung (18, 24) relativ zum Grundkörper (13) des Druckaufnehmers (12) in Richtung auf die Membran (11) verstellbar ist.

2. Druckmembrananordnung nach Anspruch 1, **dadurch gekennzeichnet**, daß der Druckaufnehmer (12) einen Anlegeteller (19) für die Membran (11) des Druckgebers (8) aufweist, wobei der Anlegeteller (19) entgegen der Ausdehnrichtung der Membran (11) federbeaufschlagt ist. 3. Druckmembrananordnung nach Anspruch 2, **dadurch gekennzeichnet**, daß der Druckschalter (22) oder Sensor im Wirkweg des Anlegetellers (19) angeordnet ist.

4. Druckmembrananordnung nach einem der Ansprüche 1 bis 3, **dadurch gekennzeichnet**, daß die Halterung (18, 24) eine Stellschraube (18) umfaßt, die in den Grundkörper (13) des Druckaufnehmers (12) einschraubbar ist.

5. Druckmembrananordnung nach Anspruch 4, **dadurch gekennzeichnet**, daß drehfest mit der Stellschraube (18) ein Stellring (24) verbunden ist, der auf seiner, dem Grundkörper (13) des Druckaufnehmers (12) zugewandten Fläche mit Ausnehmungen versehen ist, in die mittels Druckfedern (25) vorgespannte Rastkugeln (26) einführbar sind.

6. Druckmembrananordnung nach einem der Ansprüche 1 bis 5, **dadurch gekennzeichnet**, daß der Grundkörper (13) des Druckaufnehmers (12) eine Öffnung (14) mit zwei parallelen Nuten (15) aufweist, in die der Grundkörper (10) des Druckgebers (8) einsteckbar ist, wobei die mit dem Grundkörper (13) des Druckaufnehmers (12) verbundene Haltefeder (16) den Druckgeber (8) gegen Anschläge (17) im Grundkörper (13) des Druckaufnehmers (12) drückt.

7. Druckmembrananordnung .nach einem der Ansprüche 1 bis 6, **dadurch gekennzeichnet**, daß der Grundkörper (10) des Druckgebers (8) eine Ringöffnung zur Aufnahme der kreisförmigen Membran (11) aufweist.

8. Druckmembrananordnung nach einem der Ansprüche 1 bis 7, **dadurch gekennzeichnet**, daß sich in der Druckleitung (9) Luft befindet.

**Claims**

1. Pressure diaphragm arrangement for infusions, especially for infusions introduced by means of an infusion pump, with an infusion conduit (1) leading to the catheter from the container containing the infusion liquid, a closed pressure conduit (9) which is connected to the infusion conduit (1) and to whose pressure chamber there is connected a pressure transmitter (8) which has an expandable diaphragm (11), the diaphragm (11) cooperating with a pressure receiver (12) by means of which a signal emitter (28) is actuatable, and the pressure transmitter (8) having a main body (10) for supporting the diaphragm (11), which body is adapted to be inserted in a main body (13) of the pressure receiver (12), characterised in that the main body (10) of the pressure transmitter (8) is held by means of a holding spring (16) in the main body (13) of the pressure receiver (12), and that a pressure-operated switch (22) or sensor is held in an adjustable holder (18, 24) accommodated by the main body (13) of the pressure receiver (12), the holder (18, 24) being adjustable relatively to the main body (13) of the pressure receiver (12) in the direction towards the diaphragm (11).

2. Pressure diaphragm arrangement according to claim 1, characterised in that the pressure receiver (12) has an abutment plate (19) for the diaphragm (11) of the pressure transmitter (8), the abutment plate (19) being springloaded in the direction opposite to the expansion direction of the diaphragm (11).

3. Pressure diaphragm arrangement according to claim 2, characterised in that the pressure-operated switch (22) or sensor is situated in the path of action of the abutment plate (19).

4. Pressure diaphragm arrangement according to one of claims 1 to 3, characterised in that the holder (18,24) comprises an adjusting screw (18) which can be screwed into the main body (13) of the pressure receiver (12).

5. Pressure diaphragm arrangement according to claim 4, characterised in that there is connected to the adjusting screw (18), such as to be integral rotationally therewith, an adjusting ring (24) which is provided, at its surface directed towards the main body (13) of the pressure receiver (12), with recesses into which there are adapted to be introduced locking balls (26) pre-loaded by means of compression springs (25).

6. Pressure diaphragm arrangement according to one of claims 1 to 5, characterised in that the main body (13) of the pressure receiver (12) has an opening (14) with two parallel grooves (15) into which the main body (10) of the pressure transmitter (8)can be inserted,the holding spring (16), which is connected to the main body (13) of the pressure receiver (12), pressing the pressure transmitter (8) against stops (17) in the main body (13) of the pressure receiver (12).

7. Pressure diaphragm arrangement according to one of claims 1 to 6, characterised in that the main body (10) of the pressure transmitter (8) has an annular opening for receiving the circular diaphragm (11).

8. Pressure diaphragm arrangement according to one of claims 1 to 7, characterised in that air is present in the pressure conduit (9).

## Revendications

1. Dispositif à membrane à pression pour perfusions, notamment pour les perfusions introduites par une pompe à perfusion avec un tube de perfusion (1) conduisant le liquide de perfusion depuis un récipient contenant le liquide de perfusion jusqu'au cathéter, un tube de pression (9) fermé relié au tube de perfusion (1), dont la chambre de pression est reliée à un donneur de pression (8), qui présente une membrane (11) élastique, la membrane (11) coopère avec un capteur de pression (12) qui commande un émetteur de signal (28), et le donneur de pression (8) présente un corps de base (10) qui maintient la membrane (11) que l'on peut placer dans un corps de base (13) du capteur de pression (12), caractérisé en ce que le corps de base (10) du donneur de pression (8) est maintenu dans un corps de base (13) du capteur de pression (12) au moyen d'un ressort de maintien (16) et qu'un pressostat (22) ou un capteur est maintenu dans un support réglable (18, 24) qui est reçu par le corps de base (13) du capteur de pression (12), le support (18, 24) étant réglable par rapport au corps de base (13) du capteur de pression (12) dans le sens de la membrane (11).

2. Dispositif à membrane à pression selon la revendication 1, caractérisé en ce que le capteur de pression (12) présente un disque d'appui (19) pour la membrane (11) du donneur de pression (8), le disque d'appui (19) étant soumis à un ressort à l'encontre du sens de dilatation de la membrane (11).

3. Dispositif à membrane à pression selon la revendication 2, caractérisé en ce que le pressostat (22) ou le capteur est placé sur la course effective du disque d'appui (19).

4. Dispositif à membrane à pression selon l'une des revendications 1 à 3, caractérisé en ce que le support (18, 24) comprend une vis de réglage (18) que l'on peut visser dans le corps de base (13) du capteur de pression (12).

5. Dispositif à membrane à pression selon la revendication 4, caractérisé en ce qu'une bague de position (24) est solidaire de la vis de réglage (18), munie sur sa face tournée vers le corps de base (13) du capteur de pression (12) d'évidements dans lesquels on peut insérer des billes d'encliquetage (26) soumises à la contrainte de ressorts de compression (25).

6. Dispositif à membrane à pression selon l'une des revendications 1 à 5, caractérisé en ce que le corps de base (13) du capteur de pression (12) présente un alésage (14) avec deux gorges parallèles (15), dans lequel on peut insérer le corps de base (10) du donneur de pression (8), le ressort de maintien (16) relié au corps de base (13) du capteur de pression (12) appuie le donneur de pression (8) sur les butées (17) dans le corps de base (13) du capteur de pression (12).

7. Dispositif à membrane à pression selon l'une des revendications 1 à 6, caractérisé en ce que le corps de base (10) du donneur de pression (8) présente un orifice annulaire pour recevoir la membrane circulaire (11).

8. Dispositif à membrane à pression selon l'une des revendications 1 à 7, caractérisé en ce que de l'air se trouve dans le tube sous pression (9).

FIG.1

FIG.2

FIG.3

FIG.4

FIG.5

FIG.6